# EUROPÄISCHE PATENTSCHRIFT

(11) **EP 0 445 626 B1**
(45) Veröffentlichungstag und Bekanntmachung des Hinweises auf die Patenterteilung: **15.01.1997**
(21) Anmeldenummer: 91102805.8
(22) Anmeldetag: 26.02.1991
(51) Int. Cl.: G01N 33/86, C12Q 1/56

(54) **Funktioneller Test zur Bestimmung der Protein S-Aktivität**
Functional test for determining protein S activity
Test fonctionnel pour déterminer l'activité de la protéine S

(30) Priorität: 03.03.1990 DE 4006634
(43) Veröffentlichungstag der Anmeldung: 11.09.1991
(73) Patentinhaber: BEHRINGWERKE Aktiengesellschaft, 35001 Marburg (DE)
(72) Erfinder: Fickenscher, Karl, Dr., W-3550 Marburg (DE)

(56) Entgegenhaltungen:
- EP-A- 0 123 883
- EP-A- 0 158 254
- EP-A- 0 236 985
- EP-A- 0 406 971
- CLINICAL CHEMISTRY, Band 35, Nr. 8, 1989, Winston, NC (US); KOBAYASHI et al., Seiten 1644-1648/
- JOURNAL OF CLINICAL INVESTIGATION, Band 74, Dezember 1984; COMP et al., Seiten 2082-2088/
- THROMBOSIS RESEARCH, Band 48, Nr. 4, 1987; WAART et al., Seiten 427-437/
- WPIL, Week 8914, Derwent Publications Ltd., London (GB); AN 89-103936/
- WPIL, Week 8734, Derwent Publications Ltd., London (GB); AN 87-237651/

## Beschreibung

Die Erfindung betrifft ein Verfahren zur funktionellen Bestimmung von Protein S in Flüssigkeiten, insbesondere in Plasma, sowie ein hierfür geeignetes Reagenz.

Protein S ist ein Inhibitor der Blutgerinnung und wirkt als Kofaktor von aktiviertem Protein C bei dessen proteolytischem Abbau der Gerinnungsfaktoren Va und VIIIa.

Ein angeborener oder erworbener Mangel an Protein S kann zu thromboembolischen Komplikationen führen.

Protein S wird in der Leber synthetisiert (Stern, D. et al. (1986) J. Cell Biol. 102, 1971-1978). Die Biosynthese ist Vitamin K-abhängig und damit die Konzentration im Blut bei der Therapie mit Vitamin-K-Antagonisten verringert. Der Beginn einer Therapie kann, beim Vorliegen eines Protein S-Mangels, zu schweren Gesundheitsbeeinträchtigungen beim Patienten führen (Grimaudo, V. et al. (1989) BMJ 298, 233-234).

Ebenfalls verringert sein kann die Aktivität bei disseminierter intravasaler Gerinnung oder thromboembolischen Erkrankungen.

Aus den oben aufgeführten Befunden wird deutlich, daß Protein S einen entscheidenden Einfluß auf die Funktionsfähigkeit des Protein C/Thrombomodulin-Inhibitorsystems hat und ein entsprechender Bedarf für ein zuverlässiges Diagnosesystem besteht. Protein S kommt in Plasma nur zu etwa 40% in freier Form vor. Der Rest von ca. 60% befindet det sich in einem Komplex mit dem C4b- bindenden Protein (Dahlbäck, B. (1981), Proc. Natl. Acad. Sci. 78, 2512 - -2516).

Dieses gebundene Protein S ist nicht als Kofaktor für aktiviertes Protein C verfügbar und daher nicht antikoagulatorisch wirksam. Ein überhöhter Anteil gebundenes Protein S kann sich deshalb ebenfalls als ein funktioneller Mangel zeigen, während das Antigen als normal oder annähernd normal gefunden wird (Comp, P.C. et al. (1986), Blood 67, 504-508; Girolami, S. et al. (1989), Thromb. Haemost. 61, 144-147).

Andererseits kann bei erniedrigtem Protein S-Antigen die Aktivität normal sein, wenn nur ein sehr geringer Teil in der inaktiven gebundenen Form vorliegt, so wie es üblicherweise bei Neugeborenen der Fall ist (Schwarz, H.P. et al. (1988), Blood 71, 562-565).

Bekannt ist es, Protein S immunologisch zu bestimmen. Diese Methoden lassen aber keine Aussage über die Aktivität des Proteins zu und sind nicht Gegenstand der Erfindung.

Verfahren zur Bestimmung der Aktivität von Protein S sind ebenfalls beschrieben.

Die DE 3724443 A1 beschreibt ein Verfahren zur Bestimmung der Aktivität von Protein S. Dieser Test setzt relativ komplexe Reagenzien, wie ein synthetisches Substratplasma, gereinigten Faktor Xa und Prothrombin voraus und ist in der Durchführung durch die Inkubationszeiten umständlich. Er wird auch z.B. durch Heparin in der Probe gestört.

In der DE 3607559 A1 ist eine funktionelle Bestimmung von Protein S in Kombination mit Protein C erwähnt. Es ist erforderlich für jede Probe einen Bezugswert ohne Protein C Aktivator zu bestimmen. Der Einsatz eines Aktivators von F VII oder F II wird ebenfalls beansprucht, aber keine weiteren Belege aufgezeigt. Angaben über die zu erwartenden Meßwerte oder Störeinflüsse für alle Testsysteme werden nicht gemacht.

Ein funktioneller Test wird von Comp, P.C. et al. in J. Clin. Invest. 74, 2082-2088 (1984) beschrieben. Hier wird die Verlängerung der Gerinnungszeit im 1-Stufen Faktor Xa-Test durch aktiviertes Protein C gemessen. Dieser Test ist relativ insensitiv mit einer Verlängerung von nur 24 s für die mit diesem Verfahren maximale, meßbare Konzentration von 50%. Plasmen von Patienten unter Vitamin K-Antagonisten-Therapie oder mit Leberschäden waren nicht meßbar.

Bertina et al. (Thromb. Haemost. 1985, 53 (2), 268-272) zeigen eine Möglichkeit auf, funktionelles Protein S in Humanplasma zu bestimmen. Der Test ist jedoch insofern nicht quantitativ für Protein S auswertbar, da keine Kontrolle des endogenen Faktor VIII oder Faktor V im zu messenden Plasma möglich ist und von diesen beiden Faktoren die Verlängerungen entscheidend abhängen. Liegen diese Faktoren in erhöhter oder erniedrigter Konzentration in der Probe vor, so ergeben sich dementsprechend erniedrigte oder erhöhte Befunde für Protein S.

Van de Waart et al. (1987, Thromb. Res. 48, 427-437) benutzen ein System aus adsorbierten Substratplasma, zugesetztem Prothrombin, aktiviertem Protein C, Phospholipiden und Calciumchlorid. 100% Differenz im Protein S-Gehalt der Probe bewirken nur ca. 20 s Verlängerung, d.h. der Test ist ebenfalls relativ unempfindlich.

Suzuki K. und Nishioka J. (1988, Thromb. Res. 49, 241 - 251) beschreiben ein weiteres Testsystem. Sie benutzen Protac^{(R)}, einen Aktivator für Protein C aus Schlangengift. Dieses Verfahren ist ebenfalls zeitraubend und umständlich. Die Empfindlichkeit ist mit einer Verlängerung von 13 s für 100% Aktivitätsdifferenz sehr gering.

Kobayashi I. et al. (1989, Clin. Chem. 35, 1644-1648) stellen auch einen Protein S-Test vor, der ebenfalls eine Bestimmung über eine modifizierte aPTT-Bestimmung darstellt.

Den bisher bekannten Testverfahren ist gemeinsam, daß sie, neben einer umständlichen Handhabung wegen der Vielzahl der verwendeten Reagenzien nur eine geringe Sensitivität aufweisen, deren Maß die Verlängerung der Reaktionszeit in Bezug auf die Höhe der Protein S Aktivität ist.

Der Erfindung lag deshalb die Aufgabe zugrunde, ein Verfahren sowie ein Reagenz zu entwickeln, das die Aktivität von Protein S in Plasma sowohl einfach als auch zuverlässig, empfindlich und spezifisch zu bestimmen erlaubt.

Erfindungsgemäß wird nun ein Verfahren zur Bestimmung von Protein S durch die Bestimmung der Gerinnungszeit einer biologischen Probe aufgezeigt, wie in den Ansprüchen beschrieben.

Durch die Verlängerung der Gerinnungszeit konnte auf überraschend einfache Weise eine sehr große und darüberhinaus den Erfordernissen anpaßbare Empfindlichkeitssteigerung erzielt werden.

Verfahren zur Bestimmung der Gerinnungszeit sind dem Fachmann an sich bekannt. Es kann sich dabei u. a. um Verfahren handeln, die die Freisetzung von Thrombin aus Prothrombin über die Entstehung eines Gerinnsels oder die Umsetzung eines chromogenen Substrates bestimmen. Bevorzugt ist dabei das chromogene Verfahren, ganz besonders bevorzugt die Verwendung eines chromogenen Thrombinsubstrates, insbesondere von Tos-Gly-Pro-Arg-ANBA-IPA.

In einer bevorzugten Ausführung des erfindungsgemäßen Verfahrens wird die unverdünnte Probe mit einem Überschuß an Protein S Mangelplasma versetzt und dann die Gerinnungszeit durch Zumischen eines Reagenzes bestehend aus aktiviertem Protein C, einem Aktivator des exogenen oder endogenen Weges der Gerinnung, Phospholipiden, Ca++, einem chromogenen Substrat für Thrombin sowie einer Heparin neutralisierenden Substanz bestimmt. Das aktivierte Protein C kann auch separat kurz vor dem restlichen Reagenz zur Probe gegeben werden.

In einer besonders bevorzugten Ausführungsform des erfindungsgemäßen Verfahrens wird die unverdünnte Probe mit dem 4 bis 10fachen Volumen an Protein-S Mangelplasma versetzt. Die Gerinnungreaktion wird durch Zumischung (das 5 bis 10fache des Probenvolumens) eines Reagenz aus aktiviertem Protein C (1 bis 50 µmol/ml), einem Aktivator des Gerinnungssystems, bevorzugterweise ein Thromboplastin oder Sulfatid, Phospholipiden (5-300 ppm (w/v)) z. B. Cephalin, Calciumionen (2-10 mmol/l) bevorzugterweise CaCl₂, eine Heparin neutralisierende Substanz, wie z. B. Polybren (0.1-10 µg/ml) sowie ein chromogenes Thrombinsubstrat, wie z. B. Tos-Gly-Pro-Arg-ANBA-IPA. Gemessen wird die Zeit von der Zugabe des Reagenz bis zum Erreichen einer bestimmten Extinktion (z. B. 0.1) im Absorptionsoptimum (z.B. 405 nm) des freigesetzten Chromophors.

Die Aktivatorkonzentration, die unter den gegebenen Reaktionsbedingungen eine Gerinnungszeit (ohne Protein-S) von mindestens 50 s gewährleistet, kann jeweils durch einfache Versuche ermittelt werden.

Ganz besonders bevorzugt sind Verfahren und Reagenzien, die in den Beispielen beschrieben sind.

Die Gerinnungszeit wird bei der Verwendung eines chromogenen Substrates bevorzugterweise dadurch bestimmt, daß die Zeit von der Zugabe des Reagenz bis zum Erreichen einer bestimmten Extinktion im Absorptionsoptimum des freigesetzten Chromophors bestimmt wird. Zur Auswertung wird zweckmäßigerweise eine Kalibrierungskurve erstellt, indem Verdünnungen eines Poolplasmas (z.B. 100%, 75%, 50%, 25%, 12,5% 10%) in den Test eingesetzt und die Gerinnungszeiten festgestellt werden.

Die biologische Probe kann bevorzugterweise Plasma humanen Ursprungs sein, besonders bevorzugt ist dabei die Verwendung unverdünnter Proben.

Ein geeignetes Protein S-Mangelplasma kann, nach dem Fachmann an sich bekannten Verfahren, z. B. immunadsorptiv gewonnen werden. Faktor VIII wird, falls notwendig, durch Zugabe von gereinigtem Faktor VIII eingestellt. Der Faktor V kann gegebenenfalls durch Zugabe eines Protein-S Mangelplasmas aus Kaninchen, welches große Mengen an Faktor V enthält, eingestellt werden. Bevorzugterweise ist das Volumenverhältnis von Probe zu Protein S Mangelplasma 1 : 4 bis 1 : 10.

Bevorzugterweise weist das Mangelplasma einen Gehalt an Faktor V von etwa 20 bis 100 %, besonders bevorzugterweise von 50 - 80 % auf.

Protein C kann nach verschiedenen beschriebenen Verfahren aus Plasma gereinigt (z.B. Bajaj S.P. et al. (1983) Preparative Biochemistry 13(3) 191-214) oder durch biotechnologische Verfahren hergestellt werden. Das gereinigte Protein C kann mittels PROTAC^{(R)} oder Thrombin, welche an ein Trägermaterial, wie Sepharose, gekoppelt wurden, aktiviert werden oder es kann über gentechnologische Verfahren direkt gewonnenes aktiviertes Protein C (Ehrlich H.J. et al., J. Biol. Chem. 264 (24) 14298-14309) eingesetzt werden. Die Konzentration an aktiviertem Protein C im Test liegt zweckmäßigerweise zwischen 1 und 50 pmol/ml.

Bevorzugterweise kann ein, durch eine Schlangengiftprotease aus dem Gift der Agkistrodon contortrix aktiviertes Protein C eingesetzt werden.

Aktivatoren des Gerinnungssystems sind dem Fachmann an sich bekannt. Aktivatoren im Sinne dieser Erfindung können auch Schlangengiftproteasen und aktivierte Faktoren der Gerinnungskaskade, wie z. B. Faktor VIIa, IXa und Xa, sein. Bevorzugterweise können Proteasen aus dem Gift von Vipera russellii, Sulfatide, Ellagsäure, Thromboplastine und/oder Silica-Partikel eingesetzt werden (SHIMADA T. et al. (1985) J. Biochem. 97, 429 - 439). Die optimale Konzentration der jeweiligen Aktivatoren kann durch einfache Versuche ermittelt werden.

Phospholipide sind eine bekannte Klasse von Stoffen, die nach dem Fachmann an sich bekannten Verfahren hergestellt werden können oder im Handel zu erwerben sind. Bevorzugt sind Konzentrationen von 5-300 ppm (w/v) im Testansatz. Ca²⁺ Ionen können vorteilhafterweise durch Zugabe von CaCl₂ erzeugt werden. Bevorzugt sind Konzentrationen von 2 - 10 mM im Testansatz.

Heparin neutralisierende Substanzen sind eine dem Fachmann bekannte Klasse von Verbindungen, wie z. 8. Polybren, Protamin-Chlorid, Protamin-Sulfat.

Die Reaktion kann bei 15 - 40 °C, bevorzugterweise bei 20 - 40 °C, ganz bevorzugterweise bei 37 °C durchgeführt werden.

Es wurde gefunden, daß im beschriebenen Test das im Plasma vorliegende Protein S sensitiv und spezifisch gemessen werden kann (Tabelle 1). Keinen Einfluß haben Konzentrationsänderungen von Faktor VIII zwischen 50 und 150%, der Vitamin K-abhängigen Gerinnungsfaktoren wie Prothrombin oder Protein C (50-150%), außer Protein S selbst, oder die Anwesenheit von Heparin bis zu 0.4 U/ml. Der Faktor V Gehalt der Probe zeigt erwartungsgemäß einen gewissen Einfluß, da durch den Gehalt im Protein S-Mangelplasma Schwankungen in der Probe nicht völlig ausgeglichen werden können und die Inhibition dieses Faktors ein Maß für die Aktivtät des Protein S ist.

Die Erfindung betrifft ferner ein Reagenz zur Bestimmung von Protein S nach dem erfindungsgemäßen Verfahren, wobei die Gerinnungszeit einer Probe ohne Protein S mindestens 50 Sekunden beträgt.

Bevorzugt sind dabei Reagenzien, die als Aktivator Sulfatide oder Gemische aus Sulfatiden enthalten sowie Reagenzien, die als Aktivator ein Thromboplastin enthalten.

Bevorzugt sind auch Reagenzien, die ein chromogenes Thrombinsubstrat der allgemeinen Formel I enthalten 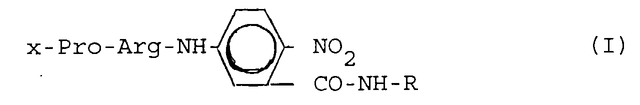 wobei
R ein Alkylrest mit 1-5 Kohlenstoffatomen oder -CH[CH(CH₃)₂]COOCH₃ und
X H-D-Phe-, Boc-Gly oder Tosyl-Gly ist.

**Tabelle 1:**

| Spezifität des Tests zur funktionellen Bestimmung von Protein S | | |
|---|---|---|
| geänderter Parameter | Konzentration | gefundene Protein S Aktivität |
| Kontrolle | | 100,0 % |
| | | |
| Heparin | 0.1 U/ml | 102 % |
| Heparin | 0.2 U/ml | 97 % |
| Heparin | 0.3 U/ml | 109 % |
| Heparin | 0.4 U/ml | 109 % |
| Heparin | 0.5 U/ml | 132 % |
| Heparin | 1.0 U/ml | > 150 % |
| | | |
| F V | 50 % | 114 % |
| F V | 75 % | 111 % |
| F V | 100 % | 100 % |
| F V | 125 % | 96 % |
| F V | 150 % | 91 % |
| | | |
| F VIII | 50 % | 106 % |
| F VIII | 75 % | 99 % |
| F VIII | 100 % | 98 % |
| F VIII | 125 % | 103 % |
| F VIII | 150 % | 104 % |
| | | |
| Protein C | 50 % | 96 % |
| Protein C | 100 % | 100 % |
| Protein C | 150 % | 103 % |

Die folgenden Beispiele dienen der Erläuterung der Erfindung und sollen sie in keiner Weise einschränken.

### Beispiel 1

### Herstellung eines gebrauchsfertigen Reagenz auf der Basis eines aPTT-Reagenzes

Zu einem aPTT-Reagenz (Partochrom^{(R)}, Behringwerke AG, D-3550 Marburg), bestehend aus Phospholipid, Sulfatid, Polybren, einem chromogenen Thrombinsubstrat (Tos-Gly-Pro-Arg-ANBA-isopropylamid) und Hepes, pH 7.6, werden 0.5 Einheiten aktiviertes Protein C und 10 µg Polybren zugegeben und das Gemisch auf 37°C erwärmt. Das Reagenz ist damit gebrauchsfertig.

### Beispiel 2

### Herstellung eines gebrauchsfertigen Reagenz auf der Basis eines PT-Reagenzes

Zu einem PT-Reagenz (Behringwerke AG, D-3550 Marburg), bestehend aus Phospholipid, einem niedrig konzentrierten Thromboplastin, einem chromogenen Thrombinsubstrat (Tos-Gly-Pro-Arg-ANBA-isopropylamid) und Hepes, pH 7.4, werden 0.5 Einheiten aktiviertes Protein C zugegeben und das Gemisch auf 37°C erwärmt. Das Reagenz ist damit gebrauchsfertig.

### Beispiel 3

### Herstellung eines gebrauchsfertigen Reagenzes auf der Basis eines Aktivators aus einem Schlagengift

Zu einem Puffer bestehend aus Phospholipid, einem chromogenen Thrombinsubstrat (Tos-Gly-Pro-Arg-ANBA-isopropylamid), einem Heparinantagonisten (Polybren), Natriumchlorid und Hepes, pH 7.0, werden 40 ng/ml eines Schlangengiftes von Vipera russellii gegeben und das Reaktionsauf 37 °C erwärmt. Das Reagenz ist damit gebrauchsfertig. Beispiel 4

### Bestimmung des Protein S-Gehalts in Plasma

### Zur Kalibrierung werden Verdünnungen eines Normalplasmas

in phosphatgepufferter, isotonischer Kochsalzlösung von 100%, 75%, 50%, 25%, 10% und 0% hergestellt. Die Messung der Standards und der Proben erfolgt wie nachstehend beschrieben: In eine Küvette werden pipettiert:
10 µl Probe
50 µl Protein S-Mangelplasma
500 µl Reagenz gemäß
   a) Beispiel 1
   b) Beispiel 2

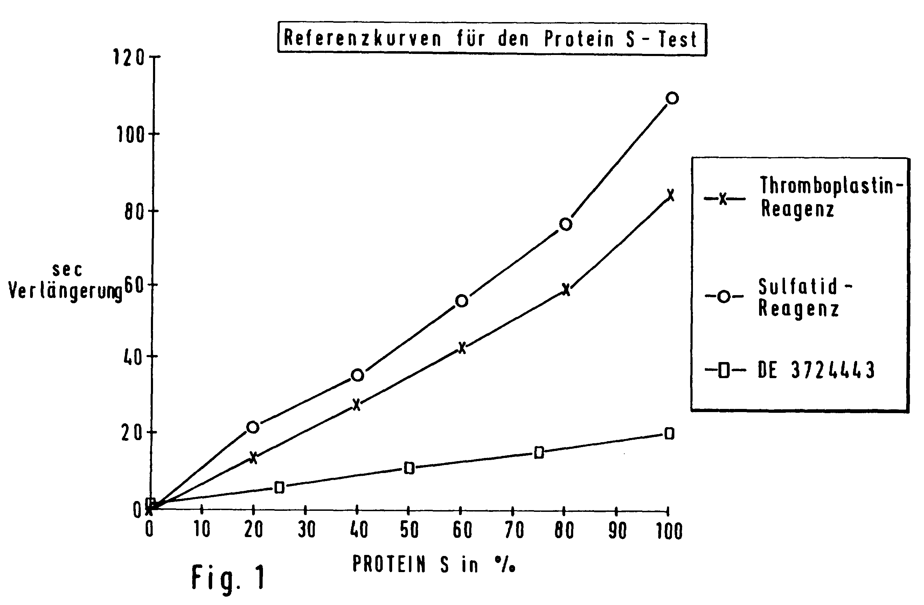

Mit der Zugabe des Reagenz wird eine Uhr gestartet und die Extinktion bei 405 nm verfolgt bis ein Anstieg in der Absorption um einen bestimmten Wert (z.B. von 0,1) erreicht worden ist. Die Verlängerung der Gerinnungszeit über den Wert für 0% Protein S hinaus ist der Konzentration an Protein S in der Probe proportional (Fig. 1).

Die Daten für die Bestimmung gemäß dem in DE 37 24 443 beschriebenen Verfahren sind der Offenlegungsschrift DE 37 24 443 A1, Figur 1, entnommen.

### Beispiel 5

### Bestimmung des Protein S-Gehalts in Plasma mit getrennter Zugabe von aktiviertem Protein C

Zur Kalibrierung werden Verdünnungen eines Normalplasmas in Saline von 100%, 75%, 50%, 25%, 10% und 0% hergestellt. Die Messung der Standards und der Proben erfolgt wie nachstehend beschrieben:

In eine Küvette werden pipettiert:
10 µl Probe
50 µl Protein S-Mangelplasma
25 µl aktiviertes Protein C
500 µl Reagenz gemäß Beispiel 3

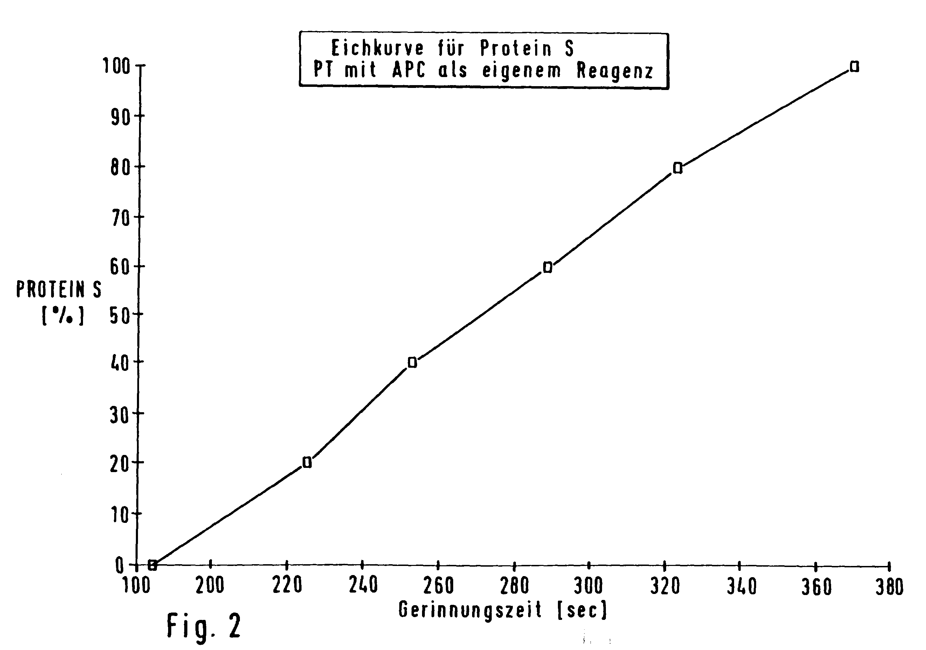

Mit der Zugabe des Reagenz wird eine Uhr gestartet und entweder die Extinktion bei 405 nm verfolgt bis ein Anstieg in der Absorption um einen bestimmten Wert (z.B. von 0,1) erreicht wird oder das Eintreten einer Gerinnselbildung gemessen. Die Verlängerung der Gerinnungszeit über den Wert für 0% Protein S hinaus ist der Konzentration an Protein S in der Probe proportional (Fig. 2).

## Patentansprüche (Patentansprüche für folgende(n) Vertragsstaat(en): AT, BE, CH, DE, DK, FR, GB, IT, LI, NL, SE)

1. Verfahren zur funktionellen Bestimmung der Protein S Aktivität in einer Probe eines biologisches Materials, das folgende Schritte einschließt:
a) die Probe eines biologischen Materials wird mit einem Überschuß an Protein S Mangelplasma versetzt,
b) mindestens ein Aktivator und aktiviertes Protein C werden zu dem
Gemisch aus Schritt a)
entweder gleichtzeitig hinzugefügt, oder
das aktivierte Protein C wird separat kurz vor dem restlichen Reagenz hinzugefügt,
c) die Gerinnungszeit wird bestimmt,
wobei die Menge des in Schritt b) hinzugefügten Aktivators so bemessen ist, daß die Gerinnungszeit in Abwesenheit von Protein S über die normale Gerinnungszeit hinaus verlängert ist.

2. Verfahren nach Anspruch 1), dadurch gekennzeichnet, daß die Gerinnungszeit in Abwesenheit von Protein S mindestens 50 s beträgt.

3. Verfahren nach Anspruch 1) oder 2) dadurch gekennzeichnet, daß die Gerinnungszeit mit Hilfe eines chromogenen Peptidsubstrats für Thrombin bestimmt wird.

4. Verfahren nach Anspruch 1) oder 2) dadurch gekennzeichnet, daß die Gerinnungszeit mit Hilfe der Gerinnselbildung bestimmt wird.

5. Verfahren nach einem der Ansprüche 1) bis 4) dadurch gekennzeichnet, daß Aktivator und Inhibitor in einem Reagenz, das mindestens Kalzium-Ionen, Phospholipide und einen Inhibitor für Heparin enthält, zugegeben wird.

6. Verfahren nach einem der Ansprüche 1) bis 4) dadurch gekennzeichnet, daß zunächst aktiviertes Protein C als Inhibitor und dann der Aktivator in einem Reagenz, das mindestens Kalzium-Ionen, Phospholipide und einen Inhibitor für Heparin enthält, zugegeben wird.

7. Verfahren nach Anspruch 3, dadurch gekennzeichnet, daß die unverdünnte Probe mit dem 4 bis 10fachen Volumen an Protein-S Mangelplasma versetzt wird, die Gerinnungsreaktion wird gestartet durch Zumischung des 5 bis 10fachen des Probenvolumens eines Reagenz aus 1 bis 50 µmol/ml aktiviertem Protein C, einem Aktivator des Gerinnungssystems 5-300 ppm (w/v) Phospholipiden (z. B. Cephalin), 2-10 mmol/l Calciumionen, einer Heparin neutralisierenden Substanz sowie einem chromogenen Thrombinsubstrat, wobei die Zeit von der Zugabe des Reagenz bis zum Erreichen einer bestimmten Extinktion im Absorptionsoptimum des freigesetzten Chromophors gemessen wird.

8. Verfahren nach Anspruch 7, wobei der Aktivator ausgewählt ist aus der Gruppe von Aktivatoren, die besteht aus Thromboplastin und Sulfatiden.

9. Verfahren nach Anspruch 7, wobei die Calciumionen in Form von CaCl₂ zugegeben werden.

10. Verfahren nach Anspruch 7, wobei die Heparin neutralisierende Substanz Polybren in einer Konzentration von 0.1 - 10 µg/ml ist.

11. Verfahren nach Anspruch 7, wobei das chromogeneThrombinsubstrat Tos-Gly-Pro-Arg-ANBA-IPA ist.

12. Verfahren nach Anspruch 1 oder 2, dadurch gekennzeichnet, daß das Mangelplasma einen Gehalt an Faktor V von 20 bis 100% aufweist.

13. Verfahren nach Anspruch 1 oder 2, dadurch gekennzeichnet, daß das Protein C durch eine Schlangengift Protease aus dem Gift der Schlange Agkistrodon contortrix aktiviert wurde.

14. Reagenz zur funktionellen Protein S - Bestimmung in einem Verfahren nach Anspruch 1), dadurch gekennzeichnet, daß es enthält: aktiviertes Protein C, einen Aktivator des exogenen oder endogenen Weges der Gerinnung, Phospholipide, Calcium-Ionen, gegebenenfalls ein chromogenes Substrat für Thrombin sowie eine Heparin neutralisierende Substanz, wobei das Verhältnis der Aktivitäten des aktivierten Protein C und des Aktivators so eingestellt ist, daß eine Gerinnungszeit in Abwesenheit von Protein S von mindestens 50 s gewährleistet ist.

15. Reagenz nach Anspruch 14, dadurch gekennzeichnet, daß der Aktivator eine Protease aus dem Gift einer Schlange oder das umfraktionierte Schlangengift ist.

16. Reagenz nach Anspruch 14, dadurch gekennzeichnet, daß als Aktivator ein Thromboplastin eingesetzt wird.

17. Reagenz nach Anspruch 14, dadurch gekennzeichnet, daß der Aktivator ein Sulfatid oder ein Gemisch aus Sulfatiden ist.

18. Reagenz nach Anspruch 14, dadurch gekennzeichnet, daß der Heparininhibitor Polybren ist.

19. Reagenz nach Anspruch 14, dadurch gekennzeichnet, daß als chromogenes Thrombinsubstrat eine Verbindung der allgemeinen Formel I eingesetzt wird 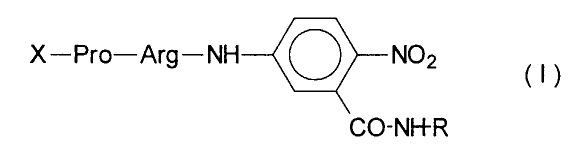 wobei
R ein Alkylrest mit 1-5 Kohlenstoffatomen oder -CH[CH(CH₃)₂]COOCH₃ und
X H-D-Phe-, Boc-Gly oder Tosyl-Gly ist.

## Patentansprüche (Patentansprüche für folgende(n) Vertragsstaat(en): ES)

1. Verfahren zur funktionellen Bestimmung der Protein S Aktivität in einer Probe eines biologisches Materials, das folgende Schritte einschließt:
a) die Probe eines biologischen Materials wird mit einem Überschuß an Protein S Mangelplasma versetzt,
b) mindestens ein Aktivator und aktiviertes Protein C werden zu dem
Gemisch aus Schritt a)
entweder gleichzeitig hinzugefügt, oder
das aktivierte Protein C wird separat kurz vor dem restlichen Reagenz hinzugefügt.
c) die Gerinnungszeit wird bestimmt,
wobei die Menge des in Schritt b) hinzugefügten Aktivators so bemessen ist, daß die Gerinnungszeit in Abwesenheit von Protein S über die normale Gerinnungszeit hinaus verlängert ist.

2. Verfahren nach Anspruch 1), dadurch gekennzeichnet, daß die Gerinnungszeit in Abwesenheit von Protein S mindestens 50 s beträgt.

3. Verfahren nach Anspruch 1) oder 2) dadurch gekennzeichnet, daß die Gerinnungszeit mit Hilfe eines chromogenen Peptidsubstrats für Thrombin bestimmt wird.

4. Verfahren nach Anspruch 1) oder 2) dadurch gekennzeichnet, daß die Gerinnungszeit mit Hilfe der Gerinnselbildung bestimmt wird.

5. Verfahren nach einem der Ansprüche 1) bis 4) dadurch gekennzeichnet, daß Aktivator und Inhibitor in einem Reagenz, das mindestens Kalzium-Ionen, Phospholipide und einen Inhibitor für Heparin enthält, zugegeben wird.

6. Verfahren nach einem der Ansprüche 1) bis 4) dadurch gekennzeichnet, daß zunächst aktiviertes Protein C als Inhibitor und dann der Aktivator in einem Reagenz, das mindestens Kalzium-Ionen, Phospholipide und einen Inhibitor für Heparin enthält, zugegeben wird.

7. Verfahren nach Anspruch 3, dadurch gekennzeichnet, daß die unverdünnte Probe mit dem 4 bis 10fachen Volumen an Protein-S Mangelplasma versetzt wird, die Gerinnungsreaktion wird gestartet durch Zumischung des 5 bis 10fachen des Probenvolumens eines Reagenz aus 1 bis 50 µmol/ml aktiviertem Protein C, einem Aktivator des Gerinnungssystems 5-300 ppm (w/v) Phospholipiden (z. B. Cephalin), 2-10 mmol/l Calciumionen, einer Heparin neutralisierenden Substanz sowie einem chromogenen Thrombinsubstrat, wobei die Zeit von der Zugabe des Reagenz bis zum Erreichen einer bestimmten Extinktion im Absorptionsoptimum des freigesetzten Chromophors gemessen wird.

8. Verfahren nach Anspruch 7, wobei der Aktivator ausgewählt ist aus der Gruppe von Aktivatoren, die besteht aus Thromboplastin und Sulfatiden.

9. Verfahren nach Anspruch 7, wobei die Calciumionen in Form von CaCl₂ zugegeben werden.

10. Verfahren nach Anspruch 7, wobei die Heparin neutralisierende Substanz Polybren in einer Konzentration von 0.1 - 10 µg/ml ist.

11. Verfahren nach Anspruch 7, wobei das chromogeneThrombinsubstrat Tos-Gly-Pro-Arg-ANBA-IPA ist.

12. Verfahren nach Anspruch 1 oder 2, dadurch gekennzeichnet, daß das Mangelplasma einen Gehalt an Faktor V von 20 bis 100% aufweist.

13. Verfahren nach Anspruch 1 oder 2, dadurch gekennzeichnet, daß das Protein C durch eine Schlangengift Protease aus dem Gift der Schlange Agkistrodon contortrix aktiviert wurde.

## Claims (Claims for the following Contracting State(s): AT, BE, CH, DE, DK, FR, GB, IT, LI, NL, SE)

1. A method for the functional determination of the protein S activity in a sample of a biological material, which includes the following steps:
a) the sample of a biological material is treated with an excess of protein S-deficient plasma,
b) at least one activator and activated protein C are either simultaneously added to the mixture from step a), or
the activated protein C is added separately shortly before the remaining reagent,
c) the clotting time is determined,
the amount of the activator added in step b) being proportioned such that the clotting time in the absence of protein S is prolonged beyond the normal clotting time.

2. The method as claimed in claim 1, wherein the clotting time in the absence of protein S is at least 50 s.

3. The method as claimed in claim 1 or 2, wherein the clotting time is determined with the aid of a chromogenic peptide substrate for thrombin.

4. The method as claimed in claim 1 or 2, wherein the clotting time is determined with the aid of clot formation.

5. The method as claimed in one of claims I to 4, wherein activator and inhibitor are added in a reagent which contains at least calcium ions, phospholipids and an inhibitor of heparin.

6. The method as claimed in one of claims 1 to 4, wherein first activated protein C is added as an inhibitor and then the activator is added in a reagent which contains at least calcium ions, phospholipids and an inhibitor of heparin.

7. The method as claimed in claim 3, wherein the undiluted sample is treated with a 4- to 10-fold volume of protein S-deficient plasma, the clotting reaction is started by adding 5 to 10-fold of the sample volume of a reagent consisting of 1 to 50 µmol/ml of activated protein C, an activator of the clotting system, 5-300 ppm (w/v) of phospholipids (e.g. cephalin), 2-10 mmol/l of calcium ions, a heparin-neutralizing substance and a chromogenic thrombin substrate, the time from the addition of the reagent until achieving a specific extinction in the absorption optimum of the released chromophore being measured.

8. The method as claimed in claim 7, the activator being selected from the group of activators which consists of thromboplastin and sulfatides.

9. The method as claimed in claim 7, the calcium ions being added in the form of CaCl₂.

10. The method as claimed in claim 7, the heparin-neutralizing substance being Polybrene in a concentration of 0.1-10 µg/ml.

11. The method as claimed in claim 7, the chromogenic thrombin substrate being Tos-Gly-Pro-Arg-ANBA-IPA.

12. The method as claimed in claim 1 or 2, wherein the deficient plasma has a content of factor V of 20 to 100%.

13. The method as claimed in claim 1 or 2, wherein the protein C was activated by a snake venom protease from the venom of the snake Agkistrodon contortrix.

14. A reagent for functional protein S determination in a method as claimed in claim 1, which contains: activated protein C, an activator of the exogenous or endogenous clotting pathway, phospholipids, calcium ions, if appropriate a chromogenic substrate for thrombin and a heparin-neutralizing substance, the ratio of the activities of the activated protein C and of the activator being adjusted such that a clotting time in the absence of protein S of at least 50 s is guaranteed.

15. The reagent as claimed in claim 14, wherein the activator is a protease from the venom of a snake or the refractionated snake venom.

16. The reagent as claimed in claim 14, wherein the activator employed is a thromboplastin.

17. The reagent as claimed in claim 14, wherein the activator is a sulfatide or a mixture of sulfatides.

18. The reagent as claimed in claim 14, wherein the heparin inhibitor is Polybrene.

19. The reagent as claimed in claim 14, wherein the chromogenic thrombin substrate employed is a compound of the general formula I 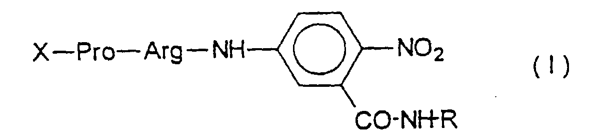 where R is an alkyl radical having 1-5 carbon atoms or -CH[CH(CH₃)₂]COOCH₃ and X is H-D-Phe-, Boc-Gly or tosyl-Gly.

## Claims (Claims for the following Contracting State(s): ES)

1. A method for the functional determination of the protein S activity in a sample of a biological material, which includes the following steps:
a) the sample of a biological material is treated with an excess of protein S-deficient plasma,
b) at least one activator and activated protein C are either simultaneously added to the mixture from step a), or
the activated protein C is added separately shortly before the remaining reagent,
c) the clotting time is determined,
the amount of the activator added in step b) being proportioned such that the clotting time in the absence of protein S is prolonged beyond the normal clotting time.

2. The method as claimed in claim 1, wherein the clotting time in the absence of protein S is at least 50 s.

3. The method as claimed in claim 1 or 2, wherein the clotting time is determined with the aid of a chromogenic peptide substrate for thrombin.

4. The method as claimed in claim 1 or 2, wherein the clotting time is determined with the aid of clot formation.

5. The method as claimed in one of claims 1 to 4, wherein activator and inhibitor are added in a reagent which contains at least calcium ions, phospholipids and an inhibitor of heparin.

6. The method as claimed in one of claims 1 to 4, wherein first activated protein C is added as an inhibitor and then the activator is added in a reagent which contains at least calcium ions, phospholipids and an inhibitor of heparin.

7. The method as claimed in claim 3, wherein the undiluted sample is treated with a 4- to 10-fold volume of protein S-deficient plasma, the clotting reaction is started by adding 5 to 10-fold of the sample volume of a reagent consisting of 1 to 50 µmol/ml of activated protein C, an activator of the clotting system, 5-300 ppm (w/v) of phospholipids (e.g. cephalin), 2-10 mmol/l of calcium ions, a heparin-neutralizing substance and a chromogenic thrombin substrate, the time from the addition of the reagent until achieving a specific extinction in the absorption optimum of the released chromophore being measured.

8. The method as claimed in claim 7, the activator being selected from the group of activators which consists of thromboplastin and sulfatides.

9. The method as claimed in claim 7, the calcium ions being added in the form of CaCl₂.

10. The method as claimed in claim 7, the heparin-neutralizing substance being Polybrene in a concentration of 0.1-10 µg/ml.

11. The method as claimed in claim 7, the chromogenic thrombin substrate being Tos-Gly-Pro-Arg-ANBA-IPA.

12. The method as claimed in claim 1 or 2, wherein the deficient plasma has a content of factor V of 20 to 100%.

13. The method as claimed in claim 1 or 2, wherein the protein C was activated by a snake venom protease from the venom of the snake Agkistrodon contortrix.

## Revendications (Revendications pour l'(les) Etat(s) contractant(s) suivant(s): AT, BE, CH, DE, DK, FR, GB, IT, LI, NL, SE)

1. Procédé pour la détermination fonctionnelle de l'activité de la protéine S dans un échantillon d'une substance biologique, comprenant les étapes suivantes:
a) on ajoute à l'échantillon d'une substance biologique un excès de plasma pauvre en protéine S,
b) soit on ajoute simultanément au moins un activateur et de la protéine C activée au mélange provenant de l'étape a), soit on y ajoute séparément la protéine C activée, peu de temps avant le réactif restant,
c) on détermine le temps de coagulation,
en choisissant la quantité de l'activateur ajouté dans l'étape b) de manière que le temps de coagulation en absence de protéine S soit prolongé par rapport au temps de coagulation normal.

2. Procédé selon la revendication 1, caractérisé en ce que le temps de coagulation en absence de protéine S est d'au moins 50 secondes.

3. Procédé selon la revendication 1 ou 2, caractérisé en ce que le temps de coagulation est déterminé à l'aide d'un substrat peptidique chromogène pour la thrombine.

4. Procédé selon la revendication 1 ou 2, caractérisé en ce que le temps de coagulation est déterminé à l'aide de la formation de caillot.

5. Procédé selon l'une des revendications 1 à 4, caractérisé en ce que l'on ajoute l'activateur et l'inhibiteur dans un réactif qui contient au moins des ions calcium, des phospholipides et un inhibiteur de l'héparine.

6. Procédé selon l'une des revendications 1 à 4, caractérisé en ce que l'on ajoute d'abord de la protéine C activée en tant qu'inhibiteur et ensuite l'activateur dans un réactif qui contient au moins des ions calcium, des phospholipides et un inhibiteur de l'héparine.

7. Procédé selon la revendication 3, caractérisé en ce que l'on ajoute à l'échantillon non dilué 4 à 10 fois son volume de plasma pauvre en protéine S, on déclenche la réaction de coagulation par addition d'un volume, représentant de 5 à 10 fois le volume de l'échantillon, d'un réactif composé de 1 à 50 µmoles/ml de protéine C activée, d'un activateur du système de coagulation, de 5-300 ppm (p/v) de phospholipides (par exemple de céphaline), de 2-10 mmoles/l d'ions calcium, d'une substance neutralisant l'héparine ainsi que d'un substrat chromogène pour la thrombine, en mesurant le temps s'écoulant entre l'addition du réactif et l'obtention d'une extinction déterminée, à l'optimum d'absorption du chromophore libéré.

8. Procédé selon la revendication 7, l'activateur étant choisi dans le groupe d'activateurs constitué par la thromboplastine et des sulfatides.

9. Procédé selon la revendication 7, dans lequel les ions calcium sont ajoutés sous forme de CaCl₂.

10. Procédé selon la revendication 7, dans lequel la substance neutralisant l'héparine est le Polybrene à une concentration de 0,1-10 µg/ml.

11. Procédé selon la revendication 7, dans lequel le substrat chromogène pour la thrombine est Tos-Gly-Pro-Arg-ANBA-IPA.

12. Procédé selon la revendication 1 ou 2, caractérisé en ce que le plasma pauvre présente une teneur en facteur V allant de 20 à 100 %.

13. Procédé selon la revendication 1 ou 2, caractérisé en ce que la protéine C a été activée par une protéase de venin de serpent, provenant du venin du serpent *Agkistrodon contortrix.*

14. Réactif pour la détermination fonctionnelle de la protéine S dans un procédé selon la revendication 1, caractérisé en ce qu'il contient: de la protéine C activée, un activateur de la voie exogène ou endogène de la coagulation, des phospholipides, des ions calcium, éventuellement un substrat chromogène pour la thrombine ainsi qu'une substance neutralisant l'héparine, le rapport des activités de la protéine C activée et de l'activateur étant ajusté de manière à garantir un temps de coagulation, en absence de protéine S, d'au moins 50 secondes.

15. Réactif selon la revendication 14, caractérisé en ce que l'activateur est une protéase provenant du venin d'un serpent ou le venin de serpent fractionné.

16. Réactif selon la revendication 14, caractérisé en ce que l'on utilise comme activateur une thromboplastine.

17. Réactif selon la revendication 14, caractérisé en ce que l'activateur est un sulfatide ou un mélange de sulfatides.

18. Réactif selon la revendication 14, caractérisé en ce que l'inhibiteur d'héparine est le Polybrene.

19. Réactif selon a revendication 14, caractérisé en ce que l'on utilise comme substrat chromogène pour la thrombine un composé de formule générale I 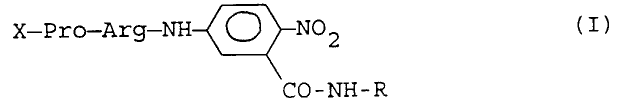 dans laquelle
R est un radical alkyle ayant de 1 à 5 atomes de carbone ou -CH[CH(CH₃)₂]COOCH₃ et
X est H-D-Phe-, Boc-Gly ou Tosyl-Gly.

## Revendications (Revendications pour l'(les) Etat(s) contractant(s) suivant(s): ES)

1. Procédé pour la détermination fonctionnelle de l'activité de la protéine S dans un échantillon d'une substance biologique, comprenant les étapes suivantes:
a) on ajoute à l'échantillon d'une substance biologique un excès de plasma pauvre en protéine S,
b) soit on ajoute simultanément au moins un activateur et de la protéine C activée au mélange provenant de l'étape a), soit on y ajoute séparément la protéine C activée, peu de temps avant le réactif restant,
c) on détermine le temps de coagulation,
en choisissant la quantité de l'activateur ajouté dans l'étape b) de manière que le temps de coagulation en absence de protéine S soit prolongé par rapport au temps de coagulation normal.

2. Procédé selon la revendication 1, caractérisé en ce que le temps de coagulation en absence de protéine S est d'au moins 50 secondes.

3. Procédé selon la revendication 1 ou 2, caractérisé en ce que le temps de coagulation est déterminé à l'aide d'un substrat peptidique chromogène pour la thrombine.

4. Procédé selon la revendication 1 ou 2, caractérisé en ce que le temps de coagulation est déterminé à l'aide de la formation de caillot.

5. Procédé selon l'une des revendications 1 à 4, caractérisé en ce que l'on ajoute l'activateur et l'inhibiteur dans un réactif qui contient au moins des ions calcium, des phospholipides et un inhibiteur de l'héparine.

6. Procédé selon l'une des revendications 1 à 4, caractérisé en ce que l'on ajoute d'abord de la protéine C activée en tant qu'inhibiteur et ensuite l'activateur dans un réactif qui contient au moins des ions calcium, des phospholipides et un inhibiteur de l'héparine.

7. Procédé selon la revendication 3, caractérisé en ce que l'on ajoute à l'échantillon non dilué 4 à 10 fois son volume de plasma pauvre en protéine S, on déclenche la réaction de coagulation par addition d'un volume, représentant de 5 à 10 fois le volume de l'échantillon, d'un réactif composé de 1 à 50 µmoles/ml de protéine C activée, d'un activateur du système de coagulation, de 5-300 ppm (p/v) de phospholipides (par exemple de céphaline), de 2-10 mmoles/l d'ions calcium, d'une substance neutralisant l'héparine ainsi que d'un substrat chromogène pour la thrombine, en mesurant le temps s'écoulant entre l'addition du réactif et l'obtention d'une extinction déterminée, à l'optimum d'absorption du chromophore libéré.

8. Procédé selon la revendication 7, l'activateur étant choisi dans le groupe d'activateurs constitué par la thromboplastine et des sulfatides.

9. Procédé selon la revendication 7, dans lequel les ions calcium sont ajoutés sous forme de CaCl₂.

10. Procédé selon la revendication 7, dans lequel la substance neutralisant l'héparine est le Polybrene à une concentration de 0,1-10 µg/ml.

11. Procédé selon la revendication 7, dans lequel le substrat chromogène pour la thrombine est Tos-Gly-Pro-Arg-ANBA-IPA.

12. Procédé selon la revendication 1 ou 2, caractérisé en ce que le plasma pauvre présente une teneur en facteur V allant de 20 à 100 %.

13. Procédé selon la revendication 1 ou 2, caractérisé en ce que la protéine C a été activée par une protéase de venin de serpent, provenant du venin du serpent *Agkistrodon contortrix.*
